# EUROPEAN PATENT APPLICATION

(11) **EP 4 678 140 A1**
(43) Date of publication of application: **14.01.2026**
(21) Application number: 23925833.8
(22) Date of filing: 30.03.2023
(51) Int. Cl.: A61C 19/04, A61B 5/00

(54) **SCANNING HEAD WITH REINFORCED ADHESIVE-BONDING STRUCTURE, AND INTRAORAL SCANNER HAVING SAME**

(30) Priority: 07.03.2023 CN 202310211929
(71) Applicant: Alliedstar Medical Equipment Co., Ltd, Ziyang, Sichuan 641300 (CN)
(72) Inventor: CHEN, Yun, Shanghai 201200 (CN); ZHENG, Yaxing, Ziyang, Sichuan 641300 (CN); LU, Hongsong, Ziyang, Sichuan 641300 (CN); WANG, Guijian, Ziyang, Sichuan 641300 (CN); LI, Hongjie, Ziyang, Sichuan 641300 (CN)
(74) Representative: Winter, Brandl - Partnerschaft mbB
(86) International application number: PCT/CN2023/085003
(87) International publication number: WO 2024/183117

(57) **Abstract**

The present disclosure belongs to the technical field of intraoral scanners, and particularly to a scanning head with a reinforced adhesive-bonding structure and an intraoral scanner that can be reused for an increased number of times and has the scanning head. The scanning head includes a plastic base layer (10) having a window formed at a tip portion thereof; an adhesive-bonding layer (20) applied to an entire or partial surface of a region of a plastic base layer directly facing the window; and a reflector layer (30) disposed within the window to be bonded to the plastic base layer (10) by the adhesive-bonding layer (20). The adhesive-bonding layer (20) is configured to enhance an embedding force with the plastic base layer (10) by a reinforcing structure so as to be strongly bonded to the reflector layer. By improving the surface energy of a plastic at a bonding site or by grafting a material with relatively high surface energy on a contact surface in an external embedding manner, and when the material externally grafted has holes and is of a reticular or porous shape, the adhesive-bonding stability is improved because an additional mechanical adhesion can be provided during permeation of an adhesive in a mesh. Alternatively, by adopting a multi-adhesive strategy, an inner adhesive guarantees a bonding force with the plastic, and an outer adhesive performs waterproofing isolation on the inner adhesive.

## Description

### FIELD

The present disclosure belongs to the technical field of intraoral scanners, and particularly to a scanning head with a reinforced adhesive-bonding structure and an intraoral scanner having the same that can be reused for an increased number of times.

### BACKGROUND

Generally, a Tip (probe) for scanning an oral cavity is a plastic product. However, because a plastic part has relatively low surface energy, the performance of a plastic changes during the high-temperature sterilization and an interactive reaction will occur between the plastic and an adhesive, a surface adhesion between a silicone and a plastic base body will decrease sharply after multiple times of high-temperature sterilization, resulting in falling off of the Tip.

In order to reduce the cost of the Tip and increase the available number of high-temperature sterilization of the Tip, the problem of the reduced strength of a bonding force between a reflector and the plastic after multiple times of high-temperature sterilization need to be solved. Such a problem exists in the industry and has not been effectively addressed.

### SUMMARY

In view of the above technical problem, an objective of the present disclosure is to provide a scanning head with a reinforced adhesive-bonding structure. An embedding force between an adhesive-bonding layer and a to-be-bonded article is increased by a reinforcing structure, the bonding strength between a reflector layer and a plastic base layer after multiple times of high-temperature sterilization is improved, and further the number of reuses of an intraoral scanner is increased.

In order to achieve the above objective, the technical solution employed by the present disclosure will be described below.

There is provided a scanning head with a reinforced adhesive-bonding structure for scanning an oral cavity, including:
a plastic base layer having a window formed at a tip portion;
an adhesive-bonding layer applied to an entire or partial surface of a region of the plastic base layer directly facing the window; and
a reflector layer disposed within the window to be bonded to the plastic base layer by the adhesive-bonding layer, wherein the adhesive-bonding layer is configured to enhance an embedding force with the plastic base layer by a reinforcing structure so as to be strongly bonded to the reflector layer.

In some embodiments, the reinforcing structure includes at least one metal mesh, and the metal mesh is embedded to a bonding region between the reflector layer and the plastic base layer, wherein the bonding region is the region of the plastic base layer directly facing the window.

In some embodiments, the metal mesh is integrally formed with the plastic base layer; or the metal mesh is connected to the plastic base layer by vacuum metal spraying, cathode coating or hot-pressing embedding.

In some embodiments, the adhesive-bonding layer includes an inner adhesive layer and an outer adhesive layer, and the inner adhesive layer is coated on an adhesive-bonding surface of the reflector layer, the outer adhesive layer is coated on an enclosing region of the plastic base layer with respect to the inner adhesive layer, and the enclosing region is positioned on the region of the plastic base layer directly facing the window.

In some embodiments, the inner adhesive layer is a liquid adhesive layer, and the outer adhesive layer is a solid adhesive layer.

In some embodiments, the inner adhesive layer is coated on a center region of the adhesive-bonding surface of the reflector layer, the outer adhesive layer is coated on a peripheral edge of the region of the plastic base layer directly facing the window, and the inner adhesive layer and the outer adhesive layer cover entirely the reflector layer when the reflector layer is bonded to the plastic base layer.

In some embodiments, the reinforcing structure includes a modified layer, wherein the modified layer is formed by changing a property of a surface of the plastic base layer.

In some embodiments, the property of the surface of the plastic base layer can be changed by heating, ion beam and laser etching.

In some embodiments, the laser etching is specifically laser engraving.

According to another aspect of the present disclosure, the present disclosure further provides an intraoral scanner that can be reused for an increased number of times, including a scanner body and the scanning head, wherein the scanner body is detachably connected to the scanning head.

The present disclosure at least has the following beneficial effects by employing the above technical solutions.
1. In the present disclosure, since the plastic has relatively low surface energy such that the adhesive-bonding structure with a reflector is easily damaged after multiple times of high-temperature sterilization, by increasing the embedding force between the adhesive-bonding layer and the plastic base layer, the bonding strength between the reflector layer and the plastic base layer is remarkably enhanced, thereby increasing the number of reuses of the intraoral scanner and reducing the cost.
2. Particularly, in the present disclosure, by changing the property of the surface of the plastic, the surface energy at the bonding site is improved, and the embedding force is increased. Alternatively, by grafting a material with relatively high surface energy on a contact surface in an external embedding manner and when the material externally grafted has holes or is of a reticular or porous shape, the adhesive-bonding stability is improved because an additional mechanical adhesion can be provided during permeation of the adhesive in a mesh. Alternatively, by adopting a multi-adhesive strategy, an inner adhesive guarantees the bonding force with the plastic, and an outer adhesive performs waterproofing isolation on the inner adhesive.

### BRIEF DESCRIPTION OF THE DRAWINGS

In order to more clearly illustrate technical solutions in embodiments of the present disclosure, figures and their reference numerals to be used in the embodiments will be briefly introduced below. It is apparent that the figures described hereinafter are only some of the embodiments of the present disclosure, and that other figures can be obtained according to these figures for those ordinarily skilled in the art without any creative effort.
FIG. 1 is a schematic view illustrating a structure of a scanning head according to a first embodiment of the present disclosure;
FIG. 2 is a schematic view illustrating a structure of a scanning head according to a second embodiment of the present disclosure;
FIG. 3 is a schematic view illustrating a structure of a scanning head according to a third embodiment of the present disclosure;
FIG. 4 is a flowchart of a metal mesh hot-pressing embedding process according to the first embodiment of the present disclosure;
FIG. 5 is a flowchart of a multi-adhesive sealing process according to the second embodiment of the present disclosure; and
FIG. 6 is a flowchart of a laser engraving process for improving the surface energy of a plastic according to the third embodiment of the present disclosure.

Meanings of reference numerals labeled in the figures will be described below:
10-plastic base layer;
20-adhesive-bonding layer, 21-inner adhesive layer, 22-outer adhesive layer;
30-reflector layer; and
41-metal mesh, and 42-modified layer.

### DETAILED DESCRIPTION OF EMBODIMENTS

In order to more clearly illustrate technical solutions in embodiments or the prior art of the present disclosure, specific embodiments of the present disclosure will be described below with reference to figures. It is apparent that the figures in the following description are only some embodiments of the present disclosure, and that other figures and other embodiments can be obtained by those ordinarily skilled in the art without any creative effort according to these figures

For the sake of simplicity of the figures, only parts related to the present disclosure are schematically shown in the figures, and do not represent their actual structures as a product. Also, in order to make the figures be simply and easily understood, in some of the figures, only one of components having the same structure or function is schematically depicted or labeled. In this text, "one" not only means "only one", but also may mean "more than one".

It should be further understood that the term "and/or" as used in the description and the appended claims of the present disclosure refers to any combination and all possible combinations of one or more of items listed in association, and includes such combinations.

In this text, it is to be noted that unless otherwise expressly specified and defined, terms "mount", "join" and "connect" should be understood broadly, for example, may be a fixed connection, or a detachable connection, or an integral connection, or a mechanical connection, or an electrical connection, or a direct connection, or an indirect connection through an intermediate medium, or a communication within two elements. Those ordinarily skilled in the art can understood specific meanings of the terms in the present disclosure as appropriate.

In the description of the present disclosure, it is to be understood that orientational or positional relationships, indicated by terms such as "center", "up", "down", "front", "back", "left", "right", "vertical", "horizontal", "top", "bottom", "inner", "outer", "axial", "radial", "circumferential", "lateral" and "longitudinal", are based on orientational or positional relationships shown in the figures and are intended only to facilitate describing the present disclosure and simplifying the description, and are not intended to indicate or imply that an apparatus or element referred to must have a particular orientation and must be constructed and operated in a specific orientation, and therefore, cannot be construed as a limitation on the present disclosure.

In the present disclosure, based on the that a plastic has relatively low surface energy such that an adhesive-bonding structure with a reflector is easily damaged after multiple times of high-temperature sterilization, an embedding force between an adhesive-bonding layer 20 and a plastic base layer 10 is improved by a reinforcing structure, the bonding strength between a reflector layer 30 and the plastic base layer 10 is significantly improved, further the number of reuses of an intraoral scanner is increased, thereby reducing cost.

### First embodiment (external embedding)

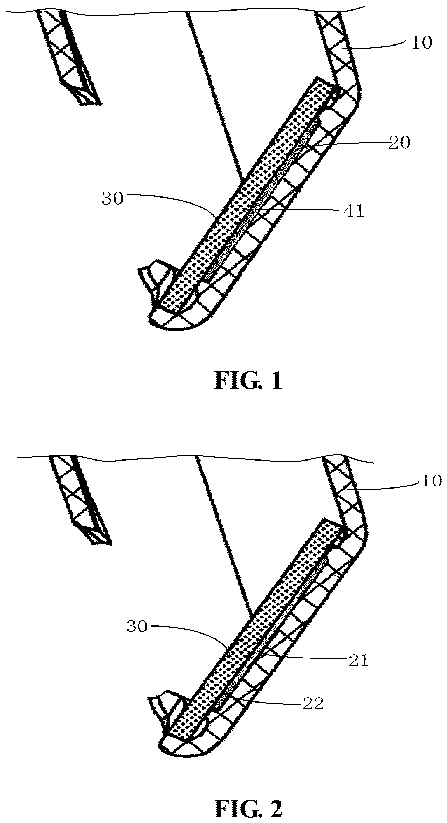

Referring to FIG. 1, there is shown a scanning head with a reinforced adhesive-bonding structure according to the present embodiment. The scanning head includes a plastic base layer 10, an adhesive-bonding layer 20 and a reflector layer 30. The plastic base layer 10 is of a shell structure, and has a window formed at a tip portion of the head. The adhesive-bonding layer 20 is applied to an entire or partial surface of a region of a plastic base layer 10directlyfacing the window. The reflector layer 30 is disposed within the window, so as to be bonded to the plastic base layer 10 by the adhesive-bonding layer 20. The adhesive-bonding layer 20 is configured to enhance an embedding force with the plastic base layer by a reinforcing structure, so as to be strongly bonded to the reflector layer. The reinforcing structure may be a metal mesh 41 embedded to a bonding region between the reflector layer 30 and the plastic base layer 10, and the bonding region is the region of the plastic base layer directly facing the window. A material with relatively high surface energy is grafted on the surface of the plastic base layer 10 by the metal mesh 41 externally embedded, such that the embedding force between an adhesive and the plastic is significantly improved, the bonding strength between the reflector layer 30 and the plastic base layer 10 is enhanced, thereby increasing the number of reuses of an intraoral scanner and reducing the cost.

The metal mesh 41 externally embedded is employed in this embodiment, on the one hand, the surface energy of the metal will be greatly improved compared to the plastic; and on the other hand, the mesh structure provides an additional mechanical adhesion for the permeation of the adhesive in the mesh, such that the adhesive-bonding stability is enhanced.

In the above embodiment, the metal mesh 41 can be directly integrally formed with the plastic as an insert in a plastic injection molding process, or can be added after the plastic injection molding process. Particularly, the metal mesh 41 can be connected to the plastic base layer 10 through vacuum metal spraying, cathodic coating or hot-pressing embedding.

In addition to simple operation, low cost and minimal thermal damage to the plastic base layer 10, a metal mesh hot-pressing embedding process will produce more stable additional mechanical connections between the metal mesh 41 and the plastic as well as between the metal mesh 41 and the adhesive after the metal mesh 41 is embedded, and this is one of more stable ways so far.

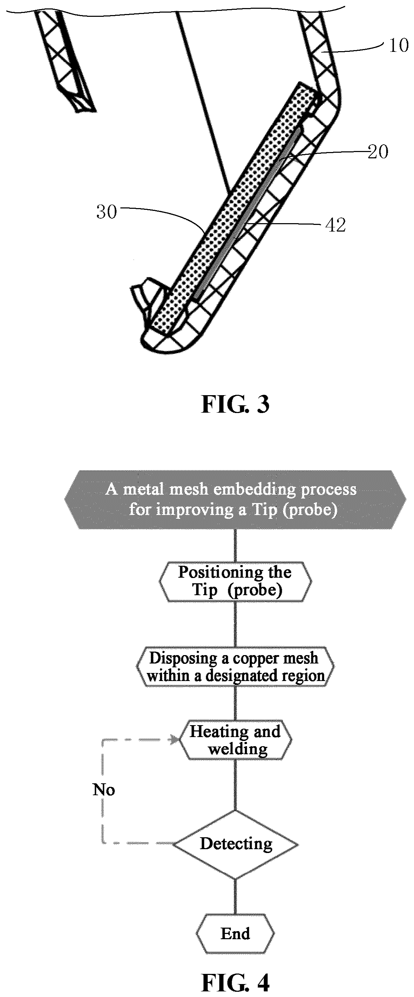

Referring to FIG. 4, there is shown a flowchart of a metal mesh hot-pressing embedding process. The metal mesh hot-pressing embedding process includes the following steps:
positioning a Tip (probe); disposing a copper mesh within a designated region; turning on a hot pressing device to ensure that a metal mesh 41 is effectively embedded to a surface of a region of a plastic base layer 10 directly facing a scanning window by using a heating and pressurizing force; performing quality detection on the fabricated scanning head, and if the quality detection fails, returning to a hot-pressing welding step, and otherwise, obtaining a qualified scanning head.

Main factors affecting the process include a material, a size and sparseness of the metal mesh 41, and a hot pressing temperature, a pressure and time of a hot pressing process.

### Second embodiment (multi-adhesive strategy)

Referring to FIG. 2, there is shown a scanning head with a reinforced adhesive-bonding structure according to the present embodiment. The scanning head includes a plastic base layer 10, an adhesive-bonding layer 20 and a reflector layer 30. The plastic base layer 10 is of a shell structure, and has a window formed at a tip portion of the head. The adhesive-bonding layer 20 is applied to an entire or partial surface of a region of a plastic base layer 10 directly facing the window. The reflector layer 30 is disposed within the window, so as to be bonded to the plastic base layer by the adhesive-bonding layer 20. The adhesive-bonding layer 20 is configured to enhance an embedding force with the plastic base layer by a reinforcing structure, so as to be strongly bonded to the reflector layer.

The adhesive-bonding layer 20 includes an inner adhesive layer 21 and an outer adhesive layer 22. The inner adhesive layer 21 is coated on an adhesive-bonding surface of the reflector layer 30, and the outer adhesive layer 22 is coated on an enclosing region of the plastic base layer 10 with respect to the inner adhesive layer 21, and the enclosing region is positioned on a region of the plastic base layer directly facing the window. By the multi-adhesive strategy, the embedding force between the adhesive and the plastic can be significantly improved, the bonding strength between the reflector layer 30 and the plastic base layer 10 is enhanced, thereby increasing the number of reuses of the intraoral scanner and reducing the cost.

In a preferred implementation, the inner adhesive layer 21 is coated on a center region of the adhesive-bonding surface of the reflector layer 30, while the outer adhesive layer 22 is coated on a peripheral edge of the region of the plastic base layer directly facing the window, and the inner adhesive layer 21 and the outer adhesive layer 22 cover the entire reflector layer 30. The maximization of the area of the bonding region can be achieved, and further the bonding strength between the reflector layer 30 and the plastic base layer 10 is enhanced.

In the above embodiment, the inner adhesive layer 21 is a liquid adhesive layer, and the outer adhesive layer 22 is a solid adhesive layer. The liquid adhesive layer guarantees the bonding force with the plastic, and the solid adhesive layer performs waterproof isolation to the inner liquid adhesive layer. A special structure of an outer adhesive layer can provide effective protection and stabilization for an inner adhesive layer to guarantee the bonding strength of the entire reflector layer 30.

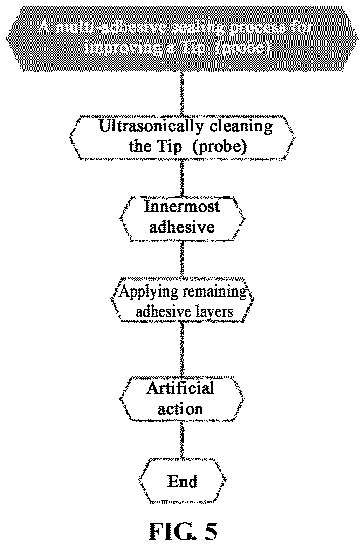

Referring to FIG. 5, there is shown a flowchart of a multi-adhesive sealing process, including the following steps:
ultrasonically cleaning a Tip (probe); applying a liquid adhesive to a center region of an adhesive-bonding surface of the reflector layer, and meanwhile, applying a solid adhesive to a peripheral edge of a plastic base layer 10 directly facing a window region; and adhesive-bonding the reflector layer to the plastic base layer 10.

Main factors affecting the process include: cleaning of a surface of a plastic, coating structures of the inner adhesive and the outer adhesive, and an adhesive-bonding temperature, humidity and time.

### Third embodiment (improvement of surface energy)

Referring to FIG. 3, there is shown a scanning head with a reinforced adhesive-bonding structure according to the present embodiment. The scanning head includes a plastic base layer 10, an adhesive-bonding layer 20 and a reflector layer 30. The plastic base layer 10 is of a shell structure, and has a window formed at a tip portion of the head. The adhesive-bonding layer 20 is applied to an entire or partial surface of a region of a plastic base layer 10 directly facing the window. The reflector layer 30 is disposed within the window, so as to be bonded to the plastic base layer 10 by the adhesive-bonding layer 20. The adhesive-bonding layer 20 is configured to enhance an embedding force with the plastic base layer by a reinforcing structure, so as to be strongly bonded to the reflector layer. The reinforcing structure includes a modified layer 42 formed by changing a property of a surface of the plastic base layer. By changing the property of the surface of the plastic, the embedding force between the adhesive and the plastic is significantly improved, the bonding strength between the reflector layer 30 and the plastic base layer 10 is enhanced, thereby increasing the number of reuses of an intraoral scanner, and reducing the cost.

In this embodiment, since the plastic has relatively low surface energy such that the adhesive-bonding structure with the reflector is easily damaged after multiple times of high-temperature sterilization, by modifying the surface of the plastic, i.e., by destroying a macromolecular structure of the plastic to fracture the macromolecular structure and form an effectively-attached micromolecule with relatively strong polarity, the surface energy of the plastic in a bonding region is directly improved.

The property of the surface of the plastic can be changed by heating, ion beam and laser etching.

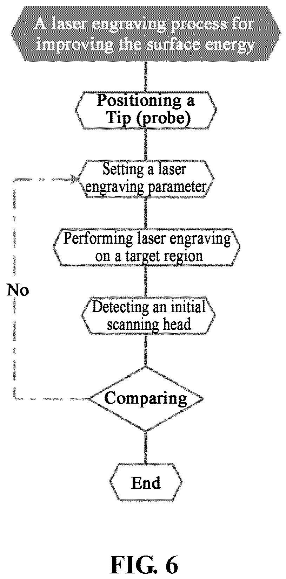

Referring to FIG. 6, there is shown a flowchart of a laser engraving process for improving the surface energy of a plastic. The laser engraving process includes the following steps:
positioning a Tip (probe); setting a laser engraving parameter; performing laser engraving on a bonding region; and performing quality detection on an initial scanning head, if the quality detection fails, then returning to reset the laser engraving parameter, and otherwise, obtaining a qualified scanning head.

Main factors affecting the process include an area and a depth of laser engraving and a power density of a laser.

The above-described embodiments present only several implementations of the present disclosure, which are described in a more specific and detailed manner, and are not construed as a limitation on the patent scope of the present disclosure. It should be noted that several variations and improvements can be made for those ordinarily skilled in the art without departing from the conception of the present disclosure, all of which fall within the scope of protection of the present disclosure. Therefore, the scope of protection of the present disclosure shall be subject to the appended claims.

Those skilled in the art should understand that although the present disclosure is described in accordance with a plurality of embodiments, not every embodiment contains only one independent technical solution. Such description in the specification is only for the sake of clarity, and those skilled in the art should understand the specification as a whole, and understand the scope of protection of the present disclosure by regarding the technical solutions involved in the embodiments as a way that the embodiments can be combined with each other to form different embodiments.

## Claims

1. A scanning head with a reinforced adhesive-bonding structure for scanning an oral cavity, comprising:
a plastic base layer having a window formed at a tip portion thereof;
an adhesive-bonding layer applied to an entire or partial surface of a region of the plastic base layer directly facing the window; and
a reflector layer disposed within the window to be bonded to the plastic base layer by the adhesive-bonding layer,
wherein the adhesive-bonding layer is configured to enhance an embedding force with the plastic base layer by a reinforcing structure so as to be strongly bonded to the reflector layer.

2. The scanning head with the reinforced adhesive-bonding structure according to claim 1, wherein:
the reinforcing structure comprises at least one metal mesh, and
the metal mesh is embedded to a bonding region between the reflector layer and the plastic base layer, wherein the bonding region is the region of the plastic base layer directly facing the window.

3. The scanning head with the reinforced adhesive-bonding structure according to claim 2, wherein:
the metal mesh is integrally formed with the plastic base layer; or
the metal mesh is connected to the plastic base layer by vacuum metal spraying, cathode coating or hot-pressing embedding.

4. The scanning head with the reinforced adhesive-bonding structure according to claim 1, wherein:
the adhesive-bonding layer comprises an inner adhesive layer and an outer adhesive layer, and
the inner adhesive layer is coated on an adhesive-bonding surface of the reflector layer, the outer adhesive layer is coated on an enclosing region of the plastic base layer with respect to the inner adhesive layer, and the enclosing region is positioned on the region of the plastic base layer directly facing the window.

5. The scanning head with the reinforced adhesive-bonding structure according to claim 4, wherein:
the inner adhesive layer is a liquid adhesive layer, and the outer adhesive layer is a solid adhesive layer.

6. The scanning head with the reinforced adhesive-bonding structure according to claim 4, wherein:
the inner adhesive layer is coated on a center region of the adhesive-bonding surface of the reflector layer, the outer adhesive layer is coated on a peripheral edge of the region of the plastic base layer directly facing the window, and the inner adhesive layer and the outer adhesive layer cover entirely the reflector layer when the reflector layer is bonded to the plastic base layer.

7. The scanning head with the reinforced adhesive-bonding structure according to claim 1, wherein:
the reinforcing structure comprises a modified layer, wherein the modified layer is formed by changing a property of a surface of the plastic base layer.

8. The scanning head with the reinforced adhesive-bonding structure according to claim 7, wherein:
the property of the surface of the plastic base layer is changed by one of heating, ion beam and laser etching.

9. The scanning head with the reinforced adhesive-bonding structure according to claim 8, wherein
the laser etching is laser engraving.

10. An intraoral scanner that can be reused for an increased number of times, comprising:
a scanner body, and
the scanning head according to any one of claims 1 to 9,
wherein the scanner body is detachably connected to the scanning head.
